# EUROPEAN PATENT SPECIFICATION

(11) **EP 4 497 426 B1**
(45) Date of publication and mention of the grant of the patent: **25.03.2026**
(21) Application number: 23187694.7
(22) Date of filing: 25.07.2023
(51) Int. Cl.: A61F 13/42, G06Q 10/087

(54) **MONITORING DEVICE ATTACHABLE TO AN ABSORBENT ARTICLE, AND RESPECTIVE METHOD FOR AUTOMATIC AND RELIABLE PRODUCT IDENTIFICATION**
AN EINEM SAUGFÄHIGEN ARTIKEL ANBRINGBARE ÜBERWACHUNGSVORRICHTUNG UND ZUGEHÖRIGES VERFAHREN ZUR AUTOMATISCHEN UND ZUVERLÄSSIGEN PRODUKTIDENTIFIZIERUNG
DISPOSITIF DE SURVEILLANCE POUVANT ÊTRE FIXÉ À UN ARTICLE ABSORBANT, ET PROCÉDÉ RESPECTIF POUR UNE IDENTIFICATION DE PRODUIT AUTOMATIQUE ET FIABLE

(43) Date of publication of application: 29.01.2025
(73) Proprietor: Ontex BV, 9255 Buggenhout (BE); Ontex Group NV, 9320 Erembodegem (BE)
(72) Inventor: PEPERMANS, Manu, 2018 Antwerpen (BE); Hellmold, Jens, 59269 Beckum (DE)
(74) Representative: Macchetta, Andrea

(56) References cited:
- EP-A1- 3 747 415
- US-A1- 2023 129 630
- US-A1- 2023 130 185
- US-A1- 2023 143 877
- US-B2- 8 421 636

## Description

### TECHNICAL FIELD

The present disclosure is directed to monitoring systems and methods for absorbent articles comprising one or more sensors for automatic exudate detection. Generally the absorbent article being a disposable personal hygiene article selected from pants, diapers and pads for incontinence. The incontinence disposable personal hygiene articles such as pants, diapers and/or pads typically being for adults i.e. adult pants, adult diapers and/or adult pads.

### BACKGROUND

Absorbent articles for personal hygiene are designed to absorb and contain bodily exudates, such as a large quantity of urine. Non-limiting examples of disposable absorbent articles include diapers, pants, training pants, pads, adult incontinence products, and feminine hygiene products (including, for example, sanitary napkins and tampons). Other examples of disposable absorbent articles include bandages and wound dressings. In some embodiments, for example, an absorbent article comprises several layers providing different functions, for example a topsheet, a backsheet and in-between an absorbent core, among other layers.

The function of the absorbent core is to absorb and retain the exudates for a prolonged amount of time, for example overnight for a diaper, minimize re-wet to keep the wearer dry and avoid soiling of clothes or bed sheets. The majority of currently marketed absorbent articles comprise as absorbent material a blend of comminuted wood pulp with superabsorbent polymers (SAP) in particulate form, also called absorbent gelling materials (AGM), see for example U.S. Pat. No. 5,151,092 (Buell). Absorbent articles having a core consisting essentially of SAP as absorbent material (so called "airfelt-free" cores) have also been proposed but are less common than traditional mixed cores (see e.g. WO2008/155699 and WO2012/052172).

A number of disclosures exist (see for example EP2496197B1, EP2739254B1, and EP2582341B1) directed to sensors to sense a condition such as temperature from body or moisture from incontinence. The sensor comprises a signal processing unit, a transmitter and a power supply, typically in form of a battery. These elements are arranged on a flexible substrate in low profile enabling disposition adjacent to the human body. A complex series of mathematical and statistical manipulations are then needed in order to determine wetness events and wetness levels.

While such devices allow monitoring conditions of the human body and can also be used as a moisture sensor, it represents also relatively costly solution. It would not be seen appropriate to dispose of the sensor together with a (disposable) absorbent article. If the sensor, however, is to be reused, the sensing area has potentially been exposed to moisture. Therefore this concept does not allow for simple usage.

Examples of articles that provide improvements to the above drawbacks are disclosed in EP3415130, WO/2018/228822, WO/2018/229017, EP3461257, and EP3451988. Further prior art is disclosed in US2023/129630, US2023/130185, US2023/143877, EP3747415 and US8421636.

A need however remains for systems and methods for improved error detection and correction related to exudate detection, particularly the location and/or amount thereof (most preferably location) within the absorbent article, and automatic product recognition and/or detection.

### SUMMARY

In a first aspect the disclosure relates to a method for monitoring at least one of exudate data and absorbent article data, wherein said exudate data is associated with at least one absorbent article and a person wearing said at least one article, and wherein said absorbent article data is associated with one or more primary article characteristics, the method comprising the steps of:
- acquiring at least one of:
   ∘ exudate data comprising exudate information for one or more voiding events, within a time period corresponding to a monitoring period, the one or more voiding events corresponding to at least a first voiding event, wherein the first, voiding event occurs at an absorbent article position; and
   ∘ absorbent article data according to the one or more primary article characteristics that are selected from the group consisting of: absorbency of said article; size of said article; construction of said article; brand of said article; material specification of said article, and combinations thereof; and
- wherein each said acquired data comprises, preferably consists of, a binary string comprising a plurality of bits that uniquely distinguishes each said acquired data; and
- identifying the presence or absence of an anomaly in the said acquired data by comparing the plurality of bits of said acquired data with bits of a plurality of pre-set binary strings comprised in a code book, each said pre-set binary strings corresponding to actual exudate and/or absorbent article data;
and wherein the method further comprises the step of carrying out a data correction step, wherein said data correction step comprises the step of replacing the acquired data corresponding to said identified anomaly with the most likely actual exudate and/or absorbent article data.

In a second aspect the disclosure relates to a data processing unit for monitoring at least one of exudate data and absorbent article data adapted to perform the method.

In a third aspect the disclosure relates to a system for monitoring at least one of exudate data and absorbent article data, the system comprising:
- a sensor associated with an absorbent article, which is arranged to generate an output signal representative of at least one of the location and/or amount of an exudate within said absorbent article, and one or more primary article characteristics of said absorbent article; and
- the data processing unit, said data processing unit being adapted to process said output signal generated by the sensor.

### BRIEF DESCRIPTION OF THE FIGURES

**FIG. 1** Is an isometric view of a device according to an embodiment of the disclosure.
**FIG. 2** Is an isometric view of a clip-on device according to an embodiment of the disclosure.
**FIG. 3** Is an plan view of an absorbent article according to an embodiment of the disclosure.
**FIG. 4** shows an example method of decoding n-bit encoded data in accordance with an embodiment of the present disclosure.
**FIG. 5** shows an example method of decoding n+2-bit encoded data in accordance with an embodiment of the present disclosure..

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's garments or undergarments when the absorbent article is worn.

As used herein, the term "absorbent article" refers to disposable devices such as infant or adult diapers or pads, pants, training pants, and the like which are placed against or in proximity to the body of the wearer to absorb and contain the various exudates discharged from the body. Typically these articles comprise a topsheet, backsheet, an absorbent core and optionally an acquisition system (which may be comprised of one or several layers) and typically other components, with the absorbent core normally placed between the backsheet and the acquisition system or topsheet.

The absorbent articles of the disclosure will be further illustrated in the below description and in the Figures, though all embodiments described herein may equally be applied onto absorbent articles in the form of pants (or even in the form of feminine hygiene articles such as menstrual pants and/or slips/panties). Nothing in this description should be however considered limiting the scope of the claims unless explicitly indicated otherwise. Unless indicated otherwise, the description refers to the dry article, i.e. before use and conditioned at least 24 hours at 21° C.+/-2° C. and 50+/-20% Relative Humidity (RH).

The terms "joined" or "associated" or "bonded" or "attached", as used herein, encompasses configurations whereby an element is directly secured to another element by affixing the element directly to the other element, and configurations whereby an element is indirectly secured to another element by affixing the element to intermediate member(s) which in turn are affixed to the other element. The terms further include embodiments in which a pocket or other connector is formed in or attached to an area of the absorbent article. Further, these terms include configurations in which the elements are removably, or non-removably, joined, bonded, or attached. For example, wherein an element is described as "joined" within the configuration, it may be either removably joined or non-removably joined unless otherwise specified or evident from the context.

The terms "comprise," "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

By "absorbent material" it is meant a material which has some absorbency property or liquid retaining properties, such as SAP, cellulosic fibers as well as synthetic fibers, most preferably is selected from the group consisting of SAP, cellulose (or cellulosic) fibers, and mixtures thereof. Herein, absorbent materials in the form of fibrous absorbent materials have been found to be useful. These fibrous absorbent materials can comprise or consist of natural fibers, e.g. cellulosic fibers as well as synthetic fibers. Typically, glues used in making absorbent cores have no absorbency properties and are not considered as absorbent material.

As used herein, the term "absorbent core" refers to the component or components of the article having the most absorbent capacity and comprising an absorbent material and optionally a core wrap enclosing the absorbent material. The term "absorbent core" does not include the acquisition-distribution system or layer or any other component of the article which is not either integral part of the core wrap or placed within the core wrap. The core may consist essentially of, or consist of, a core wrap, absorbent material as defined above and glue enclosed within the core wrap.

As used herein, "Hamming distance" between two strings of bits typically means the number of one bits in the bitwise exclusive or (XOR) of the two strings of bits.

### THE ARTICLE

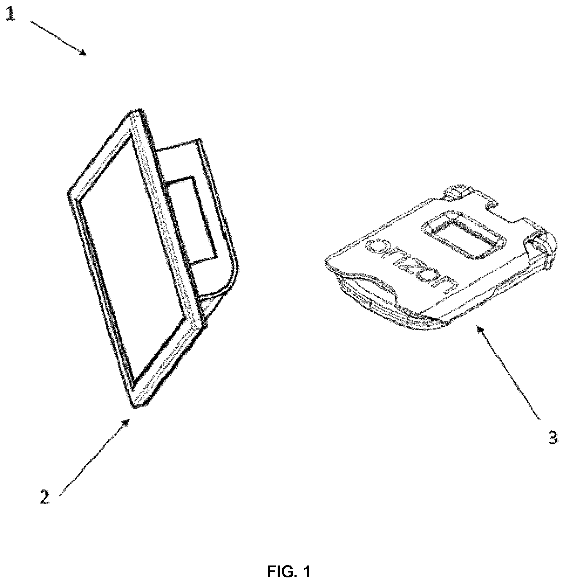

As exemplified in Fig.1 to Fig.3, the absorbent article (7) herein is generally an incontinence article selected from the group consisting of a diaper, a pant, a pad, and combinations thereof. Preferably, incontinence disposable personal hygiene articles such as pants, diapers and/or pads being for adults i.e. adult pants, adult diapers and/or adult pads.

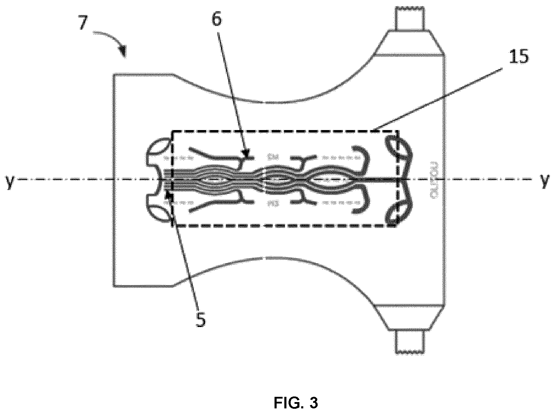

As exemplified in Fig. 3, the absorbent article (7) comprises a sensor (6) that may be applied onto a backsheet of said article. The article typically being constructed to comprise a liquid impermeable topsheet, a substantially liquid impermeable backsheet and an absorbent core (15) positioned between the topsheet and backsheet. The sensor (6) may thus be located and/or positioned on a skin facing surface of the backsheet so that at least a portion of said sensor (6) is exposed to contact with exudates upon a voiding event. The sensor (6) generally being interposed directly and/or indirectly between the absorbent core and the backsheet. The distinct layers may be joined together by construction adhesive or the like means.

The sensor (6) comprises an electrically conductive material, preferably an electrically conductive ink, having a predetermined pattern. The pattern arranged to sense the presence of exudates in different locations of the absorbent article in an x-y cartesian coordinate system, typically by a change in one or more conductivity parameters such as change in resistance and/or capacitance.

The sensor (6) comprises a plurality of connection tracks (5) wherein more than two, preferably at least 3, even more preferably from 4 to 25, of the said plurality of connection tracks (5) may be in electrical communication with a sensing pattern (or sensing tracks) comprising a plurality of open electrical circuits disposed on a portion of the article (7), preferably the backsheet thereof, and arranged such that each said circuit is able to change one or more conductivity parameters when contacted with exudates and each said circuit being representative of one or more locations of a plurality of locations of an absorbent article in an x-y cartesian coordinate system. The plurality of connection tracks (5) are preferably at least 3, preferably at least 4, more preferably from 5 to 35, connection tracks (5). Examples of suitable sensor-comprising absorbent articles for testing herein are described in EP3760104.

The sensors for use herein may thus be used to determine the location of exudates within the absorbent article (e.g. by knowing which of the plurality of circuits are triggered) but not only, for example by measuring the signal strength (e.g. resistance value) an amount of exudate (e.g. in volume, mass or the like e.g. mL, g, etc.). Exudates herein may be selected from urine, stool, menses, and combinations thereof.

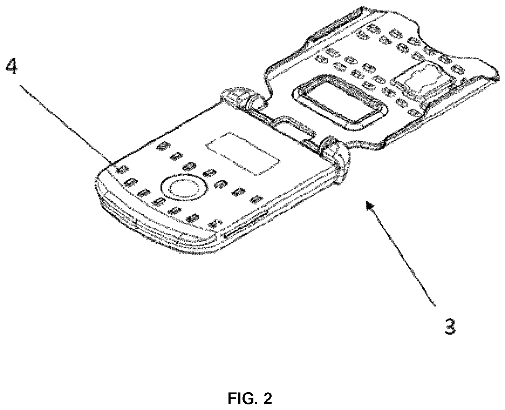

A clip-on device (3) may be connectable to the sensor (6) of the absorbent article (7). As exemplified in Fig. 2, the clip-on device (3) may comprise a power source, transmitter, a processing unit, and one or more electrical terminals (4) in electrical communication with at least one of the transmitter and the processing unit; said terminals (4) being adapted to come into contact with corresponding connection tracks (5) of a sensor (6). The clip-on device (3) may automatically acquire at least one of: exudate data comprising exudate information for one or more voiding events, preferably at least two different voiding events, within a time period corresponding to a monitoring period, the one or more voiding events corresponding to at least a first voiding event, and preferably a second voiding event, wherein the first, and preferably second, voiding event occurs at an absorbent article position [first and second voiding events can be different positions of the absorbent article]; and absorbent article data according to the one or more primary article characteristics that are selected from the group consisting of: absorbency of said article; size of said article; construction of said article; brand of said article; material specification of said article, and combinations thereof. Such data may be acquired directly to by the clip-on device (3) and transmitted directly or indirectly to a computing device (2). By indirectly it is intended herein that such data may be first transmitted to a further computing device comprising a logic and/or algorithm to process said data before being further transmitted to the computing device (2), the further computing device may be a cloud-based application.

As exemplified in Fig. 1, the computing device (2) may comprise a display, a processor and an input receiver and may be powered by a battery source or the like. The computing device (2) preferably being a hand-held device selected from the group consisting of a tablet, smartphone, portable computer, and combinations thereof.

The clip-on device (3) and the computing device (2) may thus be in direct or indirect data communication with each other. The data communication between the computing device (2) and the clip-on device (3) is preferably wireless. The wireless communication may comprise one or more of Bluetooth, Wi-fi, NFC, Zigbee, Z-Wave, and RFID. Preferably the wireless communication technology used employs radio waves and comprises frequency-hopping spread spectrum technique for secure and reliable communication. Preferably the computing device and the clip-on device are in Bluetooth wireless data communication.

The clip-on device (3) and computing device (2) may be generally referred to herein as data-capturing device or assembly (1).

### THE SYSTEM

A system for monitoring at least one of exudate data and absorbent article data herein typically comprises: a sensor (6) associated with an absorbent article (7), which is arranged to generate an output signal representative of at least one of the location (and/or amount) of an exudate within said absorbent article (7), and one or more primary article characteristics of said absorbent article (7); and a data processing unit as described herein, said data processing unit being adapted to process said output signal generated by the sensor (6).

The data processing unit for monitoring at least one of exudate data and absorbent article data is generally adapted to perform the method as described herein. The data processing unit may be comprised by the clip-on device (3), the computing device (2) and/or further computing device. Indeed activities of the data processing unit may be shared by any of the clip-on device (3), the computing device (2) and/or further computing device to limit power consumption related to the computing tasks executed, but may be equally carried out within only one or more of the clip-on device (3), the computing device (2) and further computing device such as by a processor and/or logic thereof.

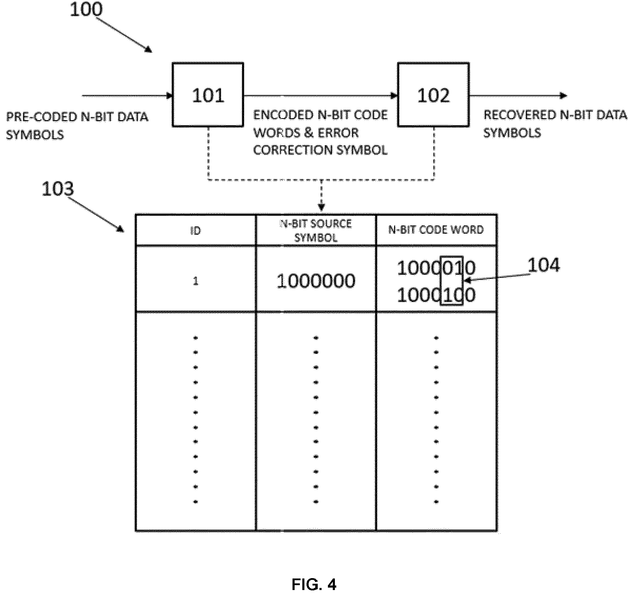

FIG. 4 illustrates an example encoding and/or decoding system 100. The system 100 may include an encoder 101 and a decoder 102. As depicted, one or more elements of the system 100 may be integrated into a single computing device and/or distributed across multiple computing devices as described herein. For example, the encoder 101 may be included in a first computing device, while the decoder 102 may be included in a second computing device, the first computing device may be the clip-on device (3) and the second computing device may be the computing device (2) or the further computing device. The first computing device may be configured to transmit data to the second computing device in accordance with any suitable wireless and/or wired protocols.

The system may comprise the article herein and adapted to carry out the method herein.

### THE METHOD

The method for monitoring at least one of exudate data and absorbent article data, wherein said exudate data is associated with at least one absorbent article and a person wearing said at least one article, and wherein said absorbent article data is associated with one or more primary article characteristics, comprises the steps of:
- acquiring at least one of:
   **∘** exudate data comprising exudate information for one or more voiding events, preferably at least two different voiding events, within a time period corresponding to a monitoring period, the one or more voiding events corresponding to at least a first voiding event, and preferably a second voiding event, wherein the first, and preferably second, voiding event occurs at an absorbent article position, wherein the first and second voiding events can be different positions of the absorbent article as typically explained herein; and
   ∘ absorbent article data according to the one or more primary article characteristics that are selected from the group consisting of: absorbency of said article; size of said article; construction of said article; brand of said article; material specification of said article, and combinations thereof; and
- wherein each said acquired data comprises, preferably consists of, a binary string comprising a plurality of bits that uniquely distinguishes each said acquired data; and
- identifying the presence or absence of an anomaly in the said acquired data by comparing the plurality of bits of said acquired data with bits of a plurality of pre-set binary strings comprised in a code book, each said pre-set binary strings corresponding to actual exudate and/or absorbent article data;
and wherein the method further comprises the step of carrying out a data correction step, preferably for each identified anomaly, wherein said data correction step comprises replacing the acquired data corresponding to said identified anomaly with the most likely actual exudate and/or absorbent article data. Advantageously this allows no gaps in data flow e.g. an inexistent product ID being reported; and/or an automated logical assessment of which circuit within a plurality of circuits of the sensor are actually triggering the signal. This whilst ensuring power containment and minimising use of energy.

The at least first voiding event may comprise a first amount of exudate and wherein the acquired exudate data may comprise information related to said first amount of exudate. The second voiding event may comprise a second amount of exudate and wherein the acquired exudate data may comprise information related to said second amount of exudate, wherein the first and second amounts may be the same or different.

The step of identifying the presence or absence of an anomaly may comprise using a statistical or computational model for determining outliers from one or more acquired exudate and/or absorbent article data, wherein said statistical or computational model is selected from k-NN, z-score, normal distribution, LOF, isolation forests, Bayesian network, neural network, decision tree, and combinations thereof. Preferably the statistical or computational model being selected from is selected from k-NN, z-score, Bayesian network, neural network, and combinations thereof. Advantageously, the identification of an anomaly in this manner allows for an increased level of accuracy of determination that may allow for more subtle determinations compared to some other alternative methods that are described.

The step of identifying the presence of an anomaly comprises the step of determining that the binary string of the acquired exudate data and/or absorbent article data is different from any of the pre-set binary strings of the plurality of pre-set binary strings; and/or wherein the code book comprises the pre-set binary strings and sequences and/or data pattern in which the said binary strings are expected for the acquisition of data. For example, if the acquired data comprises a string of 7 bits being 1000000 and when comparing to pre-set binary strings there is no sequential match of each bit in the string, an anomaly may be recognised.

The correction step may comprise the steps of determining the Hamming distance between the, preferably each of the, binary string of the acquired exudate and/or absorbent article data and a plurality of the pre-set binary strings; and outputting as the actual exudate and/or absorbent article data a value associated with the pre-set binary string having a minimum said Hamming distance. For example, with reference to Fig. 4, each of the binary strings 1000010 and 1000100 are at a Hamming distance of 1 compared to the binary string 1000000.

Preferably, the correction step comprises the step of outputting an indication that no acquired exudate and/or absorbent article data are detected when the minimum Hamming distance is greater than a given threshold, preferably wherein said threshold is a Hamming distance of 3 or more. In this embodiment, it may also be possible to automatically replace the respective acquired data with historical data for example relating to the absorbent article size or absorbency previously used or worn by the subject (or wearer) or for example relating to historical voiding patterns of the subject (or wearer). Advantageously, the risk of false information being transmitted is removed since at higher Hamming distances the potential for error increases exponentially given the possible permutations in assessing the most likely actual data that would be necessary, this in turn allows to reduce computing power and energy and/or time usage whilst limiting impacts of the removal of such data point from a plurality of data points by treating it as an outlier.

Preferably, the correction step comprises the step of combining known parameters of the person wearing said at least one article and two or more pre-set binary strings having an equal minimum Hamming distance to determine the most likely pre-set binary string corresponding to the actual exudate and/or absorbent article data, preferably by excluding all but one of said pre-set binary strings. Preferably wherein the know parameters are selected from the group of weight, age, sex, medical condition such as incontinence type or profile of the wearer/subject, historical data (such as wetness pattern of the wearer; absorbent article information, such as one or more primary article characteristics as described herein, either previously used or worn by the wearer or stocked in a nursing home), and combinations thereof. Advantageously this allows to easily exclude e.g. product IDs associated with a bit string/binary string having the same Hamming distance to others but yet cannot be the correct for example because representing a size that does not fit the wearer, or the absorbency level required by the wearer in view of its medical condition and so on.

The absorbent article data may be subsequently used in stock management and/or inventory management related to a plurality of absorbent articles held within an institution selected from hospitals and nursing homes. Advantageously this allows for operational simplification of the product management activities within a nursing home or institution.

Referring to Fig. 4, The method may comprise the step of encoding a data set including one or more n-bit pre-coded symbols within an encoder of a computing system. Preferably wherein the method comprises determining a plurality of n-bit code words, each of the plurality of n-bit code words having two or less Hamming distance from one another; mapping each of the plurality of n-bit code words to a corresponding source symbol; receiving the one or more n-bit pre-coded symbols at the encoder; matching each n-bit pre-coded symbol to a corresponding n-bit code word based on the mapping to produce encoded data; and outputting the encoded data, wherein each said code word corresponds to the binary string of a plurality of bits.

For example, pre-coded data symbols having n bits each may be input into the encoder 101. The encoder 101 generates and/or consults a table 103 responsive to receiving the pre-coded data symbols. For example, the table 103 may be a lookup table of valid n-bit code words. The encoder 101 may generate encoded data including encoded n-bit code words based on the table 106. The encoder 101 and the decoder 102 may be configured to process data in accordance with a processing pipeline illustrated in FIG. 4. For example, pre-coded data symbols having n bits each may be input into the encoder 101. The encoder 101 generates and/or consults a table 103 responsive to receiving the pre-coded data symbols. For example, the table 103 may be a lookup table of valid n-bit code words. The encoder 101 may generate encoded data including encoded n-bit code words based on the table 106.

The method may comprise the step of outputting an error correction symbol, the error correction symbol particular to the encoded data, preferably wherein the error correction symbol comprises a checksum for the encoded data and/or wherein the error correction symbol comprises a cyclic redundancy check (CRC) symbol for the encoded data or an XOR sum of bits of the encoded data.

In an embodiment, the wireless communication or transmission may be bi-directional and comprises the use of LoRa and generally comprising long range radio communication typically based on spread spectrum modulation techniques derived from chirp spread spectrum (CCS).

Preferably, each of the plurality of code words includes at most n consecutive bits of the same value, wherein n is less than 10, preferably less than 7, even more preferably from 1 to 5.

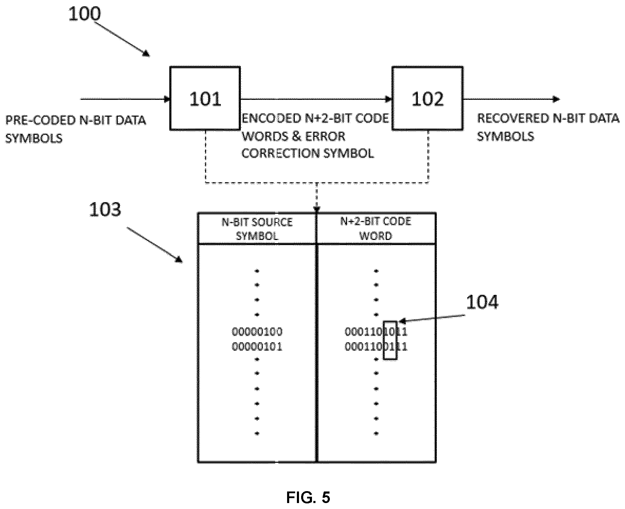

In an embodiment, and exemplified in Fig. 5, the encoder 101 and the decoder 102 may be configured to process data in accordance with the process-flow pipeline illustrated in FIG. 5. For example, pre-coded data symbols having n bits each may be input into the encoder 101. The encoder 101 generates and/or consults a table 103 responsive to receiving the pre-coded data symbols. For example, the table 103 may be a lookup table of valid n+2-bit code words. The encoder 101 may generate encoded data including encoded n+2-bit code words based on the table 103. The encoder 101 and the decoder 102 may be configured to process data in accordance with the process-flow pipeline illustrated in FIG. 5. For example, pre-coded data symbols having n bits each may be input into the encoder 101. The encoder 101 generates and/or consults a table 103 responsive to receiving the pre-coded data symbols. For example, the table 103 may be a lookup table of valid n+2-bit code words. The encoder 101 may generate encoded data including encoded n+2-bit code words based on the table 103.

As shown in FIG. 5, the table 103 may include each valid n+2-bit code word for each n-bit source symbol. Each n-bit source symbol may be matched to one or more n+2- bit code words such that more than one n+2-bit code words may correspond to the same source symbol. During encoding, the encoder 101 may match a particular pre-coded n-bit data symbol to an n-bit source symbol and output an n+2-bit code word corresponding to the n-bit source symbol. For example, as shown in the table 103, a pre-coded n-bit data symbol of 00000100 may be associated with an n+2-bit code word of 0001101011. Therefore, the encoder 101 may output 0001101011 in response to receiving a pre-coded symbol of 00000100. In some embodiments, the encoder 101 may append or otherwise include one or more error correction symbols for the encoded data. The error correction symbols may include checksum information, cyclic redundancy check information, and/or any other suitable error correction information, as described in more detail below.

The decoder 102 may receive the encoded data, consult the table 103 and/or a local version of the table 103, and output recovered n-bit data symbols. Accordingly, the decoder 102 may match a received or otherwise input encoded n+2-bit code word to the n+2-bit code words in the table 103, then output an n-bit source symbol corresponding to the n+2-bit code word as the recovered n-bit data symbol. In contrast to the matching of source symbols to code words, any n+2-bit code word may be matched with only one n-bit source symbol in order to prevent uncertainty during decoding. Using the example above, if the decoder 102 receives the code word 0001101011 from the encoder 101, the decoder 102 may output 00000100 as the recovered n-bit data symbol. The decoder 102 may utilize the one or more error correction symbols from the encoder 101 to ensure that the recovered n-bit data symbols correctly represent and/or match the pre-coded n-bit data symbols.

The scenario described above assumes an error-free transmission, in which the decoder 102 receives the exact same symbol output by the encoder 101. In some scenarios, however, transmission errors may occur in which a received symbol does not directly correspond to a transmitted symbol. For example, errors may occur at various locations of a system, such as an input and/or output of an element or within the transmission medium. In order to detect and subsequently correct such errors, the encoder 101 may be configured to determine a plurality of code words such that each code word includes one or more, preferably at least two, different bits from each other code word. The determined code words may be generated by the encoder or received from an external table generator. This difference is referred to as a Hamming distance and is illustrated at box 104 of FIG. 5. As shown in box 104, the seventh and eighth bits (reading left to right) of the first code word are each different from the seventh and eighth bits of the second code word. All other bits are the same. As such, the illustrated example N+2-bit code words have a hamming distance equal to two.

By determining a list of valid code words each having a Hamming distance of two or greater from one another, a single bit error that occurs during transmission of a valid code word may be detected as an invalid code word at a receiving element. For example, the encoder 101 may output the code word 000110011, but an error may cause the code word 0001101111 to be received at the decoder 102. The code word 0001101111 would not be a valid code word, because it has a Hamming distance of 1 from the valid code word 0001100111. When the decoder 102 consults the table 103, the received code word will not be located. Accordingly, the decoder may mark the code word as a bad symbol, such that it may be corrected. Once the code word is marked as a bad symbol, higher layers of the system may be configured to correct the error. The error detection based on receiving an invalid code word may allow the system to utilize more simplistic error correction mechanisms than systems that do not perform such error detection.

Further considerations for determining valid n+2-bit code words may be included to maintain and/or increase performance parameters in comparison to other encoding methods. For example, the encoding of the system 100 may adhere to some standards of 8b/10b encoding schemes. In some embodiments, the valid code words may have a bounded disparity of ones and zeros such that the number of ones and zeros sent over a period of time is substantially equal and the transmission is DC balanced. Some source symbols may be associated with two valid code words. Selection of a code word for a particular pre-coded symbol matching one of these source symbols may be performed based on the code words that have been transmitted prior to encoding the particular pre- coded symbol. For example, if a running disparity of the transmission is negative, in which the number of zeros transmitted is greater than the number of ones, the code word having more ones than zeros may be selected to correspond to the particular pre-coded symbol.

The valid n+2-bit code words may also be selected to have no more than a maximum number of consecutive ones and/or a maximum number of consecutive zeros in order to achieve a particular edge rate. Receiving adjacent bits with different logical values (e.g., toggling from one to zero) allows a system to perform clock synchronization and correct drift that may be experienced after receiving a string of bits having the same logical value. Therefore, the valid n+2-bit code words may be configured to match a suitable edge rate. For example, the n+2-bit code words may include no more than six consecutive bits of the same logical value to achieve an edge rate of 1/6. As shown in table 103, each of the example code words have at most 3 consecutive bits of the same logical value - 3 zeros and 3 ones in the first code word and 3 zeros in the second code word. It is appreciated that the encoder and/or table generator may select code words configured to achieve any suitable edge rate; however a number of possible valid n+2-bit code words decreases as the edge rate decreases.

The n+2-bit code words may also be selected such that a particular number of control codes is available. For example, when encoding 8-bit pre-coded symbols with 10- bit code words, 270 symbols may be represented by the code words. As the 8-bit pre- coded symbols have 256 possible combinations, 256 of the 270 code words may be reserved for representing data, while the remaining 14 symbols may represent control codes. Control codes may include any suitable code for controlling an aspect of the encoding and/or decoding system, including but not limited to start-of-frame, end-of- frame, etc.

The method may include correcting the n+2-bit code word based on the error correction symbol. In accordance with the Open Systems Interconnection (OSI) model, a computing device may be segmented into stacked layers that each serve an immediately higher layer and are served by an immediately lower layer. The error detection may be performed at any suitable element or layer of a computing device including a decoder. For example, the error detection may be performed at a physical, or lowest, layer of infrastructure of the computing device, such that the invalid symbol is marked when received at the physical layer. However, further or subsequent error correction may be performed by a higher layer than the physical layer, such as the link layer. The higher layer may utilize the error correction symbol to perform forward error correction such that the symbol may be recovered without resending the symbol. Since the symbol that includes a single-bit error is known, checksum symbols, CRC symbols, and/or XOR-based single-symbol corruption correction schemes may be utilized to correct the symbol marked as invalid or "bad". For example, in XOR-based single-symbol corruption schemes, an XOR sum of bits of the encoded data may be determined and transmitted as a parity bit. When received, the receiver may reconstruct a corrupted data symbol marked invalid or "bad" by the physical layer by: modifying the stream by replacing the corrupted data symbol with a 0 bit, calculating the new XOR parity on the modified stream, and XOR-ing the locally computed parity with the received parity. The result of the XOR operation is the recovered data symbol. Therefore, given the information in the error correction symbol and the knowledge of the "bad" symbol, the upper layer of the computing device may be able to recover the n-bit data symbols from the encoded data.

Although reference is made herein to one or more primary article characteristics, it is understood that the absorbent article data may be further associated with and/or to one or more secondary article characteristics, one or more tertiary article characteristics, and so on.., and may be adopted in methods herein described. For example, one or more secondary article characteristics may be selected from a new article and a used article; the one or more tertiary article characteristics may be selected from connection and no connection of the clip-on device to the absorbent article sensor; and the like.

## Claims

1. A method for monitoring at least one of exudate data and absorbent article data, wherein said exudate data is associated with at least one absorbent article and a person wearing said at least one article, and wherein said absorbent article data is associated with one or more primary article characteristics, the method comprising the steps of:
- acquiring at least one of:
∘ exudate data comprising exudate information for one or more voiding events within a time period corresponding to a monitoring period, the one or more voiding events corresponding to at least a first voiding event wherein the first voiding event occurs at an absorbent article position; and
∘ absorbent article data according to the one or more primary article characteristics that are selected from the group consisting of: absorbency of said article; size of said article; construction of said article; brand of said article; material specification of said article, and combinations thereof; and
- wherein each said acquired data comprises a binary string comprising a plurality of bits that uniquely distinguishes each said acquired data; **characterised in that** the method comprises the step of:
- identifying the presence or absence of an anomaly in the said acquired data by comparing the plurality of bits of said acquired data with bits of a plurality of pre-set binary strings comprised in a code book, each said pre-set binary strings corresponding to actual exudate and/or absorbent article data;
and **in that** the method further comprises the step of carrying out a data correction step, wherein said data correction step comprises the step of replacing the acquired data corresponding to said identified anomaly with the most likely actual exudate and/or absorbent article data.

2. A method according to Claim 1 wherein the construction of said article is selected from the group consisting of a diaper, a pant, a pad, and combinations thereof.

3. A method according to any of the preceding Claims wherein the step of identifying the presence or absence of an anomaly comprises using a statistical or computational model for determining outliers from one or more acquired exudate and/or absorbent article data, wherein said statistical or computational model is selected from k-NN, z-score, Bayesian network, neural network, and combinations thereof.

4. A method according to any of the preceding Claims wherein the step of identifying the presence of an anomaly comprises the step of determining that the binary string of the acquired exudate data and/or absorbent article data is different from any of the pre-set binary strings of the plurality of pre-set binary strings; and/or wherein the code book comprises the pre-set binary strings and sequences and/or data pattern for which the said binary strings are expected to be for the said acquisition of data.

5. A method according to any of the preceding Claims wherein the correction step comprises the step of determining the Hamming distance between the, preferably each of the, binary string of the acquired exudate and/or absorbent article data and a plurality of the pre-set binary strings; and outputting as the actual exudate and/or absorbent article data a value associated with the pre-set binary string having a minimum said Hamming distance.

6. A method according to Claim 5 wherein the correction step comprises the step of outputting an indication that no acquired exudate and/or absorbent article data are detected when the minimum Hamming distance is greater than a given threshold, preferably wherein said threshold is a Hamming distance of 3 or more.

7. A method according to any of Claims 5 to 6 wherein the correction step comprises the step of combining known parameters of the person wearing said at least one article and two or more pre-set binary strings having an equal minimum Hamming distance to determine the most likely pre-set binary string corresponding to the actual exudate and/or absorbent article data, preferably by excluding all but one of said pre-set binary strings.

8. A method according to Claim 7 wherein the know parameters are selected from the group of weight, age, sex, medical condition, historical data, and combinations thereof.

9. A method according to any of the preceding Claims wherein absorbent article data is subsequently used in stock management and/or inventory management related to a plurality of absorbent articles held within an institution selected from hospitals and nursing homes.

10. A method according to any of the preceding Claims comprising the step of encoding a data set including one or more n-bit pre-coded symbols within an encoder of a computing system.

11. A method according to Claim 10 wherein the method comprises determining a plurality of n-bit code words, each of the plurality of n-bit code words having two or less Hamming distance from one another; mapping each of the plurality of n-bit code words to a corresponding source symbol; receiving the one or more n-bit pre-coded symbols at the encoder; matching each n-bit pre-coded symbol to a corresponding n-bit code word based on the mapping to produce encoded data; and outputting the encoded data, wherein each said code word corresponds to the binary string of a plurality of bits.

12. A method according to any one of Claims 10 to 11 comprising the step of outputting an error correction symbol, the error correction symbol particular to the encoded data, preferably wherein the error correction symbol comprises a checksum for the encoded data and/or wherein the error correction symbol comprises a cyclic redundancy check (CRC) symbol for the encoded data or an XOR sum of bits of the encoded data.

13. A method according to any one of Claims 10 to 12 wherein each of the plurality of code words includes at most n consecutive bits of the same value, wherein n is less than 10, preferably less than 7, even more preferably from 1 to 5.

14. A data processing unit for monitoring at least one of exudate data and absorbent article data adapted to perform the method according to any one of Claims 1 to 13.

15. A system for monitoring at least one of exudate data and absorbent article data, the system comprising:
- a sensor associated with an absorbent article, which is arranged to generate an output signal representative of at least one of the location and/or amount of an exudate within said absorbent article, and one or more primary article characteristics of said absorbent article; and
- a data processing unit according to Claim 14, said data processing unit being adapted to process said output signal generated by the sensor.

## Patentansprüche

1. Verfahren zum Überwachen von mindestens einem von Exsudatdaten und Absorptionsartikeldaten, wobei die Exsudatdaten mit mindestens einem Absorptionsartikel und einer Person, die den mindestens einen Artikel trägt, assoziiert sind, und wobei die Absorptionsartikeldaten mit einer oder mehreren primären Artikeleigenschaften assoziiert sind, das Verfahren umfassend die Schritte:
- Erfassen von mindestens einem von:
∘ Exsudatdaten, umfassend Exsudatinformationen für ein oder mehrere Entleerungsereignisse innerhalb einer Zeitspanne, die einer Überwachungsperiode entspricht, wobei das eine oder die mehreren Entleerungsereignisse mindestens einem ersten Entleerungsereignis entsprechen, wobei das erste Entleerungsereignis an einer Absorptionsartikelposition auftritt; und
∘ Absorptionsartikeldaten gemäß der einen oder den mehreren primären Artikeleigenschaften, die aus der Gruppe ausgewählt sind, die zusammengesetzt ist aus: Absorptionsfähigkeit des Artikels; Größe des Artikels; Konstruktion des Artikels; Marke des Artikels; Materialspezifikation des Artikels und Kombinationen davon; und
wobei jede der erfassten Daten eine binäre Zeichenfolge umfasst, umfassend eine Vielzahl von Bits, die jede erfasste Daten eindeutig unterscheidet;
**dadurch gekennzeichnet, dass** das Verfahren den Schritt umfasst:
- Identifizieren des Vorhandenseins oder Fehlens einer Anomalie in den erfassten Daten durch Vergleichen der Vielzahl von Bits der erfassten Daten mit Bits einer Vielzahl von voreingestellten binären Zeichenfolgen, die in einem Codebuch enthalten sind, wobei jede der voreingestellten binären Zeichenfolgen tatsächlichen Exsudat- und/oder Absorptionsartikeldaten entspricht;
und **dadurch, dass** das Verfahren ferner den Schritt eines Ausführens eines Datenbehebungsschritts umfasst, wobei der Datenbehebungsschritt den Schritt eines Ersetzens der erfassten Daten, die der identifizierten Anomalie entsprechen, durch die wahrscheinlichsten tatsächlichen Exsudat- und/oder Absorptionsartikeldaten umfasst.

2. Verfahren nach Anspruch 1, wobei die Konstruktion des Artikels aus der Gruppe ausgewählt ist, bestehend aus einer Windel, einer Hose, einer Einlage und Kombinationen davon.

3. Verfahren nach einem der vorstehenden Ansprüche, wobei der Schritt des Identifizierens des Vorhandenseins oder Fehlens einer Anomalie ein Verwenden eines statistischen oder rechnerischen Modells zum Bestimmen von Ausreißern aus einem oder mehreren erfassten Exsudat- und/oder Absorptionsartikeldaten umfasst, wobei das statistische oder rechnerische Modell aus k-NN, z-Score, Bayes'schem Netzwerk, neuronalem Netzwerk und Kombinationen davon ausgewählt ist.

4. Verfahren nach einem der vorstehenden Ansprüche, wobei der Schritt des Identifizierens des Vorhandenseins einer Anomalie den Schritt des Bestimmens umfasst, dass die binäre Zeichenfolge der erfassten Exsudatdaten und/oder Absorptionsartikeldaten sich von einer beliebigen der voreingestellten binären Zeichenfolgen der Vielzahl von voreingestellten binären Zeichenfolgen unterscheidet; und/oder wobei das Codebuch die voreingestellten binären Zeichenfolgen und Sequenzen und/oder Datenmuster umfasst, für die die binären Zeichenfolgen für die Datenerfassung erwartet werden.

5. Verfahren nach einem der vorstehenden Ansprüche, wobei der Behebungsschritt den Schritt des Bestimmens der Hamming-Distanz zwischen der, vorzugsweise jeder der, binären Zeichenfolge der erfassten Exsudat- und/oder Absorptionsartikeldaten und einer Vielzahl der voreingestellten binären Zeichenfolgen umfasst; und Ausgeben als die tatsächlichen Exsudat- und/oder Absorptionsartikeldaten eines Werts, der mit der voreingestellten binären Zeichenfolge assoziiert ist, die eine minimalen Hamming-Distanz aufweist.

6. Verfahren nach Anspruch 5, wobei der Behebungsschritt den Schritt des Ausgebens einer Anzeige umfasst, dass keine erfassten Exsudat- und/oder Absorptionsartikeldaten erfasst werden, wenn die minimale Hamming-Distanz größer als ein gegebener Schwellenwert ist, vorzugsweise wobei der Schwellenwert eine Hamming-Distanz von 3 oder mehr ist.

7. Verfahren nach einem der Ansprüche 5 bis 6, wobei der Behebungsschritt den Schritt eines Kombinierens bekannter Parameter der Person, die den mindestens einen Artikel trägt, und zwei oder mehr voreingestellte binäre Zeichenfolgen umfasst, die eine gleiche minimale Hamming-Distanz aufweisen, um die wahrscheinlichste voreingestellte binäre Zeichenfolge zu bestimmen, die den tatsächlichen Exsudat- und/oder Absorptionsartikeldaten entspricht, vorzugsweise durch Ausschließen aller bis auf eine der voreingestellten binären Zeichenfolgen.

8. Verfahren nach Anspruch 7, wobei die bekannten Parameter aus der Gruppe von Gewicht, Alter, Geschlecht, Gesundheitszustand, historischen Daten und Kombinationen davon ausgewählt sind.

9. Verfahren nach einem der vorstehenden Ansprüche, wobei Absorptionsartikeldaten anschließend in der Bestandsverwaltung und/oder Lagerverwaltung in Bezug auf eine Vielzahl von Absorptionsartikeln verwendet werden, die innerhalb einer Institution gehalten werden, die aus Krankenhäusern und Pflegeheimen ausgewählt ist.

10. Verfahren nach einem der vorstehenden Ansprüche, umfassend den Schritt des Codierens eines Datensatzes, der ein oder mehrere n-Bit-vorcodierte Symbole einschließt, innerhalb eines Codierers eines Rechensystems.

11. Verfahren nach Anspruch 10, wobei das Verfahren das Bestimmen einer Vielzahl von n-Bit-Codewörtern umfasst, wobei jedes der Vielzahl von n-Bit-Codewörtern zwei oder weniger Hamming-Distanz voneinander aufweist; Zuordnen jedes der Vielzahl von n-Bit-Codewörtern zu einem entsprechenden Quellsymbol; Empfangen der einen oder mehreren n-Bit-vorcodierten Symbole an dem Codierer; Zuordnen jedes n-Bit-vorcodierten Symbols zu einem entsprechenden n-Bit-Codewort basierend auf der Abbildung, um codierte Daten zu erzeugen; und Ausgeben der codierten Daten, wobei jedes Codewort der binären Zeichenfolgen einer Vielzahl von Bits entspricht.

12. Verfahren nach einem der Ansprüche 10 bis 11, umfassend den Schritt des Ausgebens eines Fehlerbehebungssymbols, wobei das Fehlerbehebungssymbol spezifisch für die codierten Daten ist, vorzugsweise wobei das Fehlerbehebungssymbol eine Prüfsumme für die codierten Daten umfasst und/oder wobei das Fehlerbehebungssymbol ein zyklisches Redundanzprüfungssysmbol (CRC-Symbol) für die codierten Daten oder eine XOR-Summe von Bits der codierten Daten umfasst.

13. Verfahren nach einem der Ansprüche 10 bis 12, wobei jedes der Vielzahl von Codewörtern höchstens n aufeinanderfolgende Bits desselben Werts einschließt, wobei n kleiner als 10, vorzugsweise kleiner als 7, noch mehr bevorzugt von 1 bis 5, ist.

14. Datenverarbeitungseinheit zum Überwachen von mindestens einem von Exsudatdaten und Absorptionsartikeldaten, die angepasst ist, um das Verfahren nach einem der Ansprüche 1 bis 13 durchzuführen.

15. System zum Überwachen von mindestens einem von Exsudatdaten und Absorptionsartikeldaten, das System umfassend:
- einen Sensor, der mit einem Absorptionsartikel assoziiert ist, der angeordnet ist, um ein Ausgangssignal zu erzeugen, das repräsentativ für mindestens eines von der Lage und/oder Menge eines Exsudats innerhalb des Absorptionsartikels und eine oder mehrere primäre Artikeleigenschaften des Absorptionsartikels ist; und
- eine Datenverarbeitungseinheit nach Anspruch 14, wobei die Datenverarbeitungseinheit angepasst ist, um das Ausgangssignal zu verarbeiten, das durch den erzeugt wird.

## Revendications

1. Procédé de surveillance d'au moins l'une parmi des données d'exsudat et des données d'article absorbant, dans lequel lesdites données d'exsudat sont associées à au moins un article absorbant et à une personne portant ledit au moins un article et dans lequel lesdites données d'article absorbant sont associées à une ou plusieurs caractéristiques primaires d'article, le procédé comprenant les étapes consistant à :
- acquérir au moins l'une parmi :
∘ des données d'exsudat comprenant des informations d'exsudat pour un ou plusieurs événements de miction dans une période de temps correspondant à une période de surveillance, le ou les événements de miction correspondant à au moins un premier événement de miction, dans lequel le premier événement de miction se produit à une position d'article absorbant ; et
∘ des données d'article absorbant en fonction de la ou des caractéristiques primaires d'article qui sont sélectionnées dans le groupe constitué de : pouvoir absorbant dudit article ; taille dudit article ; construction dudit article ; marque dudit article ; spécification de matériau dudit article et leurs combinaisons ; et
- dans lequel chaque dite donnée acquise comprend une chaîne binaire comprenant une pluralité de bits qui distingue de manière unique chaque dite donnée acquise ; **caractérisé en ce que** le procédé comprend l'étape consistant à :
- identifier la présence ou l'absence d'une anomalie dans lesdites données acquises par comparaison de la pluralité de bits desdites données acquises à des bits d'une pluralité de chaînes binaires prédéfinies comprises dans un livre de codes, chacune desdites chaînes binaires prédéfinies correspondant à des données réelles d'exsudat et/ou d'article absorbant ;
et **en ce que** le procédé comprend en outre l'étape consistant à effectuer une étape de correction de données, dans lequel ladite étape de correction de données comprend l'étape consistant à remplacer les données acquises correspondant à ladite anomalie identifiée par les données réelles les plus probables d'exsudat et/ou d'article absorbant.

2. Procédé selon la revendication 1, dans lequel la construction dudit article est choisie dans le groupe constitué d'une couche, d'une culotte, d'une serviette et de leurs combinaisons.

3. Procédé selon l'une quelconque des revendications précédentes, dans lequel l'étape consistant à identifier la présence ou l'absence d'une anomalie comprend l'utilisation d'un modèle statistique ou informatique pour la détermination de valeurs aberrantes parmi une ou plusieurs données acquises d'exsudat et/ou d'article absorbant, dans lequel ledit modèle statistique ou informatique est choisi parmi kPPV, cote Z, réseau bayésien, réseau neuronal et leurs combinaisons.

4. Procédé selon l'une quelconque des revendications précédentes, dans lequel l'étape consistant à identifier la présence d'une anomalie comprend l'étape consistant à déterminer que la chaîne binaire des données acquises d'exsudat et/ou d'article absorbant est différente de l'une quelconque des chaînes binaires prédéfinies de la pluralité de chaînes binaires prédéfinies ; et/ou dans lequel le livre de codes comprend les chaînes binaires prédéfinies et des séquences et/ou motifs de données pour lesquels lesdites chaînes binaires sont attendues pour ladite acquisition de données.

5. Procédé selon l'une quelconque des revendications précédentes, dans lequel l'étape de correction comprend l'étape consistant à déterminer la distance de Hamming entre la chaîne binaire, de préférence chacune des chaînes binaires, des données acquises d'exsudat et/ou d'article absorbant et une pluralité de chaînes binaires prédéfinies ; et à délivrer en sortie, en tant que données réelles d'exsudat et/ou d'article absorbant, une valeur associée à la chaîne binaire prédéfinie ayant ladite distance de Hamming minimale.

6. Procédé selon la revendication 5, dans lequel l'étape de correction comprend l'étape consistant à délivrer en sortie une indication selon laquelle aucune donnée acquise d'exsudat et/ou d'article absorbant n'est détectée lorsque la distance de Hamming minimale est supérieure à un seuil donné, de préférence dans lequel ledit seuil est une distance de Hamming de 3 ou plus.

7. Procédé selon l'une quelconque des revendications 5 à 6, dans lequel l'étape de correction comprend l'étape consistant à combiner des paramètres connus de la personne portant ledit au moins un article et deux chaînes binaires prédéfinies ou plus ayant une distance de Hamming minimale égale afin de déterminer la chaîne binaire prédéfinie la plus probable correspondant aux données réelles d'exsudat et/ou d'article absorbant, de préférence par exclusion de toutes les chaînes binaires prédéfinies sauf une.

8. Procédé selon la revendication 7, dans lequel les paramètres connus sont choisis dans le groupe constitué du poids, de l'âge, du sexe, de l'état de santé, des antécédents médicaux et de leurs combinaisons.

9. Procédé selon l'une quelconque des revendications précédentes, dans lequel les données d'article absorbant sont ensuite utilisées dans la gestion des stocks et/ou des inventaires liée à une pluralité d'articles absorbants détenus au sein d'un établissement choisi parmi les hôpitaux et les maisons de retraite.

10. Procédé selon l'une quelconque des revendications précédentes, comprenant l'étape consistant à coder un ensemble de données comportant un ou plusieurs symboles précodés de n bits à l'intérieur d'un codeur d'un système informatique.

11. Procédé selon la revendication 10, dans lequel le procédé comprend la détermination d'une pluralité de mots de code de n bits, chacun de la pluralité de mots de code de n bits ayant une distance de Hamming de deux ou moins les uns par rapport aux autres ; la mise en correspondance de chacun de la pluralité de mots de code de n bits avec un symbole source correspondant ; la réception du ou des symboles précodés de n bits au niveau du codeur ; la mise en correspondance de chaque symbole précodé de n bits avec un mot de code de n bits correspondant, sur la base de la mise en correspondance, afin de produire des données codées ; et la délivrance en sortie des données codées, dans lequel chaque dit mot de code correspondant à la chaîne binaire d'une pluralité de bits.

12. Procédé selon l'une quelconque des revendications 10 à 11, comprenant l'étape consistant à délivrer en sortie un symbole de correction d'erreur, le symbole de correction d'erreur étant particulier aux données codées, de préférence dans lequel le symbole de correction d'erreur comprend une somme de contrôle pour les données codées et/ou dans lequel le symbole de correction d'erreur comprend un symbole de contrôle de redondance cyclique (CRC) pour les données codées ou une somme XOR de bits des données codées.

13. Procédé selon l'une quelconque des revendications 10 à 12, dans lequel chacun de la pluralité de mots de code comporte au plus n bits consécutifs de la même valeur, où n est inférieur à 10, de préférence inférieur à 7, encore plus préférablement de 1 à 5.

14. Unité de traitement de données pour la surveillance d'au moins l'une parmi des données d'exsudat et des données d'article absorbant, adaptée pour effectuer le procédé selon l'une quelconque des revendications 1 à 13.

15. Système de surveillance d'au moins l'une parmi des données d'exsudat et des données d'article absorbant, le système comprenant :
- un capteur associé à un article absorbant, qui est agencé pour la génération d'un signal de sortie représentatif d'au moins l'un parmi l'emplacement et/ou la quantité d'un exsudat à l'intérieur dudit article absorbant et d'une ou de plusieurs caractéristiques primaires dudit article absorbant ; et
- une unité de traitement de données selon la revendication 14, ladite unité de traitement de données étant adaptée pour traiter ledit signal de sortie généré par le capteur.
